# EUROPEAN PATENT APPLICATION

(11) **EP 3 995 166 A1**
(43) Date of publication of application: **11.05.2022**
(21) Application number: 20834571.0
(22) Date of filing: 02.07.2020
(51) Int. Cl.: A61M 11/04, A61M 15/00, H05B 1/02, A24F 40/50, A24F 40/57, G06F 11/07

(54) **PORTABLE AEROSOL GENERATOR AND OPERATION METHOD THEREOF**

(30) Priority: 02.07.2019 KR 20190079354; 15.07.2019 KR 20190085162
(71) Applicant: Em-tech. Co., Ltd., Changwon-si, Gyeongsangnam-do 51539 (KR)
(72) Inventor: SON, Joo Ho, Seoul 03633 (KR); LEE, Hee Jun, Seoul 04056 (KR); JUNG, Jin Woo, Anyang-si Gyeonggi-do 13991 (KR)
(74) Representative: Müller Hoffmann & Partner
(86) International application number: PCT/KR2020/008654
(87) International publication number: WO 2021/002698

(57) **Abstract**

The present invention relates to a portable aerosol generating device which generates aerosol by vaporizing an inhalation material, and more particularly, to a portable aerosol generating device capable of preventing overheating and/or equipment failure by sensing an error in a heating operation and controlling the heating operation. The portable aerosol generating device according to an embodiment of the present invention comprises: a heater for generating heat as it is supplied with current; a battery which can supply instantaneous high power to the heater; a heater control circuit for regulating power supplied from the battery to the heater; a microcontroller for controlling the operation of the heater control circuit for a heating time; and an error control means provided separately from the microcontroller and connected to both the microcontroller and the heater control circuit to receive and count a control signal being transferred from the microcontroller to the heater control circuit and simultaneously control the operation of the heater control circuit depending on the counting result.

## Description

### TECHNICAL FIELD

The present invention relates to a portable aerosol generating device which generates aerosol by vaporizing an inhalation material, and more particularly, to a portable aerosol generating device capable of preventing overheating and/or equipment failure by sensing an error in a heating operation and controlling the heating operation.

### BACKGROUND ART

Aerosols are small liquid or solid particles suspended in the atmosphere that typically have a size of 0.001 to 1.0 *µ*m. In particular, people may inhale aerosols derived from various types of liquids for various purposes. For example, a nebulizer is known for treating diseases.

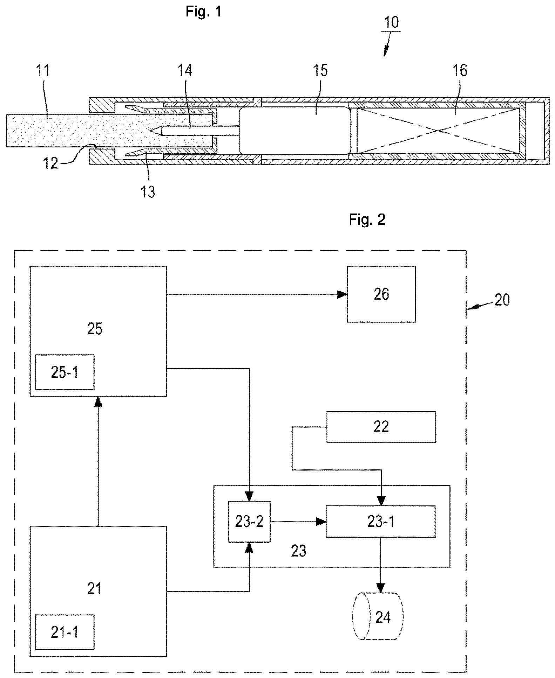

FIG. 1 is a schematic sectional view showing an example of a conventional portable aerosol generating device. Referring to FIG. 1, the conventional portable aerosol generating device 10 includes a heater 14 for generating heat by a resistance as it is supplied with current, an electricity storage unit 16 which can supply instantaneous high power to the heater 14, and a microcontroller 15 for controlling the heater 14. The heater 14 generates aerosol by heating a vaporizable member housed in a cavity 13, the vaporizable member containing a material that vaporizes when heated above a certain temperature. For example, when an aerosol-forming substrate 11 filled with paper covered on its surface with an inhalation material or impregnated with the same is inserted into the cavity 13 through an opening 12, the heater 14 is heated to vaporize the inhalation material inside the aerosol-forming substrate 11, such that the user can inhale the inhalation material vaporized through a filter part of the aerosol-forming substrate 11. In the conventional portable aerosol generating device 10 described above, a timer or counter is built in the microcontroller 15, such that the microcontroller 15 controls a heating time of the heater 14 by counting the timer or counter according to the preset heating time and controls a heating operation of the heater 14 by forwarding a control signal. Since there is no structure or method for providing against a malfunction of the timer or counter or for sensing a malfunction of the microcontroller 15, if the microcontroller 15 or the timer or counter built-in the microcontroller 15 malfunctions, the heater 14 may be overheated, which causes a safety problem. In addition, the Korean registered patent publication number 10-1824765 discloses an aerosol-generating device having a reusable heating element. However, it does not describe a structure and method capable of preventing overheating of the aerosol-generating device by sensing a heating time error in a heating operation or a malfunction of a microcontroller.

### DISCLOSURE OF THE INVENTION

The present invention has been conceived to solve the foregoing problems, and an object of the present invention is to provide a portable aerosol generating device and its operation method capable of preventing overheating and/or equipment failure by preventing an error in a heating operation.

Another object of the present invention is to provide a portable aerosol generating device and its operation method capable of preventing overheating and/or equipment failure by sensing a malfunction of a microcontroller in a heating operation and controlling the heating operation.

According to an aspect of the present invention, there is provided a portable aerosol generating device comprising an error control means provided separately from a microcontroller and connected to both the microcontroller and a heater control circuit to receive and count a control signal being transferred from the microcontroller to the heater control circuit and simultaneously control the operation of the heater control circuit depending on the counting result.

In some embodiments, the microcontroller and the error control means may comprise timers, respectively, and the error control means may receive a control signal based on the timer of the microcontroller, compare the control signal with a control signal based on the timer of the error control means, and send to the heater control circuit the control signal based on the timer independently from the microcontroller.

In some embodiments, the error control means may comprise a D/A converter for receiving a control signal transferred from the microcontroller to the heater control circuit and converting the control signal into an analog signal, a comparator for comparing the signal output from the D/A converter with a reference value and forwarding an output signal, and a timer for counting the output signal forwarded from the comparator.

In some embodiments, the error control means may comprise a frequency counter for receiving a control signal transferred from the microcontroller to the heater control circuit and counting its frequency and a timer for counting a count time of the frequency counter.

The present invention makes it possible to provide a portable aerosol generating device capable of preventing overheating of a heater by dual-controlling a heating time by an error control means provided separately from a microcontroller.

In addition, the present invention makes it possible to a portable aerosol generating device capable of preventing overheating of a heater and/or equipment failure by including an error control means which can determine a PWM signal of a microcontroller and control a heater control circuit.

### BRIEF DESCRIPTION OF THE DRAWINGS

FIG. 1 is a schematic sectional view showing an example of a conventional portable aerosol generating device.
FIG. 2 is a block diagram for explaining a portable aerosol generating device according to one embodiment of the present invention.
FIG. 3 is a block diagram for explaining a portable aerosol generating device according to another embodiment of the present invention.
FIG. 4 is a block diagram for explaining a portable aerosol generating device according to a further embodiment of the present invention.
FIG. 5 is a flowchart for explaining an operation method of a portable aerosol generating device according to the present invention.
FIG. 6 is another flowchart for explaining the operation method of the portable aerosol generating device according to the present invention.

### BEST MODE FOR CARRYING OUT THE INVENTION

A portable aerosol generating device according to one embodiment of the present invention comprises a heater for generating heat as it is supplied with current, a battery which can supply instantaneous high power to the heater, a heater control circuit for regulating power supplied from the battery to the heater, a microcontroller for controlling the operation of the heater control circuit for a heating time, and an error control means provided separately from the microcontroller and connected to both the microcontroller and the heater control circuit to receive and count a control signal being transferred from the microcontroller to the heater control circuit and simultaneously control the operation of the heater control circuit depending on the counting result.

In some embodiments, the microcontroller and the error control means may comprise timers, respectively, and the error control means may receive a control signal based on the timer of the microcontroller, compare the control signal with a control signal based on the timer of the error control means, and send to the heater control circuit the control signal based on the timer independently from the microcontroller.

In some embodiments, the error control means may comprise a D/A converter for receiving a control signal transferred from the microcontroller to the heater control circuit and converting the control signal into an analog signal, a comparator for comparing the signal output from the D/A converter with a reference value and forwarding an output signal, and a timer for counting the output signal forwarded from the comparator.

In some embodiments, the error control means may comprise a frequency counter for receiving a control signal transferred from the microcontroller to the heater control circuit and counting its frequency and a timer for counting a count time of the frequency counter.

In some embodiments, the control signal based on the timer of the microcontroller may be a PWM signal transferred from the microcontroller to the heater control circuit.

In some embodiments, the heater control circuit may comprise a FET connected between the battery and the heater and connected to the microcontroller and a switching unit connected between the microcontroller and the FET and connected to the error control means to be turned on and off under the control of the error control means.

In some embodiments, the portable aerosol generating device may comprise a power supply unit turned on and off under the control of the error control means.

In addition, an operation method of a portable aerosol generating device according to one embodiment of the present invention comprises the steps of: starting heating; performing the heating; counting a control signal based on a timer of a microcontroller; determining the counting result; and controlling the heating depending on the counting result.

In some embodiments, the operation method may further comprise a step of counting a control signal based on a timer of an error control means, in combination with the step of counting the control signal based on the timer of the microcontroller.

In some embodiments, in the step of counting the control signal based on the timer of the microcontroller, the control signal based on the timer of the microcontroller may be a PWM signal and a frequency of the PWM signal may be counted.

In some embodiments, in the step of determining the counting result, whether the heating time has been completed may be determined.

In some embodiments, in the step of counting the control signal based on the timer of the microcontroller, the control signal based on the timer of the microcontroller may be a PWM signal and a duty duration of the PWM signal may be counted, and in the step of determining the counting result, the duty duration may be compared with the preset time.

In some embodiments, in the step of controlling the heating depending on the counting result, a switching unit of a heater control circuit may be turned on and off depending on the counting result.

In some embodiments, in the step of controlling the heating depending on the counting result, a power supply unit may be turned on and off depending on the counting result.

Hereinafter, a portable aerosol generating device and its operation method according to preferred embodiments of the present invention will now be described in detail with reference to the attached drawings.

FIG. 2 is a block diagram for explaining a portable aerosol generating device according to one embodiment of the present invention. Referring to FIG. 2, the portable aerosol generating device 20 according to one embodiment of the present invention includes a heater 24 for generating heat as it is supplied with current, a battery 22 which can supply instantaneous high power to the heater 24, a heater control circuit 23 for regulating power supplied from the battery 22 to the heater 24, a microcontroller 21 for controlling the operation of the heater control circuit 23 for a heating time, and an error control means 25 provided separately from the microcontroller 21 and connected to both the microcontroller 21 and the heater control circuit 23 to receive and count a control signal being transferred from the microcontroller 21 to the heater control circuit 23 and simultaneously control the operation of the heater control circuit 23 depending on the counting result. When the heating starts, the microcontroller 21 forwards a control signal to the heater control circuit 23, and power supplied from the battery 22 is regulated in the heater control circuit 23 and supplied to the heater 24. In some embodiments, the microcontroller 21 and the error control means 25 include timers 21-1 and 25-1, respectively, and the error control means 25 receives a control signal based on the timer 21-1 of the microcontroller 21 and compares the control signal with a control signal based on the timer 25-1 of the error control means 25. For example, the control signal based on the timer 21-1 of the microcontroller 21 is a PWM signal, and the error control means 25 counts a frequency of the PWM signal input from the microcontroller 21 and also counts a frequency of the control signal based on the timer 25-1 of the error control means 25, counting a heating time. If the frequency of the PWM signal input from the microcontroller 21 is not counted for the preset heating time, the error control means 25 sends a control signal to the heater control circuit 23 to perform the heating operation for the preset heating time. In addition, if the frequency of the PWM signal input from the microcontroller 21 is counted beyond the preset heating time, the error control means 25 sends a control signal to the heater control circuit 23 to stop the heating operation. During the heating operation, the duty of the PWM signal is changed to maintain the varying temperature. In some embodiments, the error control means 25 compares a duty duration of the PWM signal input from the microcontroller 21 with the preset time and if the duty duration is abnormal, the error control means 25 sends a control signal to the heater control circuit 23 to stop the heating operation. In some embodiments, the heater control circuit 23 includes a FET 23-1 connected between the battery 22 and the heater 24 and connected to the microcontroller 21 and a switching unit 23-2 connected between the microcontroller 21 and the FET 23-1 and connected to the error control means 25 to be turned on and off under the control of the error control means 25. Here, in case of controlling the heating operation to be performed, the error control means 25 sends a control signal to the FET 23-1 through the switching unit 23-2 of the heater control circuit 23 to perform the heating operation for the preset heating time. Further, in case of controlling the heating operation to be stopped, the error control means 25 sends a control signal to the switching unit 23-2 of the heater control circuit 23 to turn off the switching unit 23-2 to stop the heating operation. In some embodiments, the portable aerosol generating device includes a power supply unit 26 turned on and off under the control of the error control means 25, and the error control means 25 may stop the heating operation by controlling the power supply unit 26. In some embodiments, the respective timers 21-1 and 25-1 may adopt the respective counters.

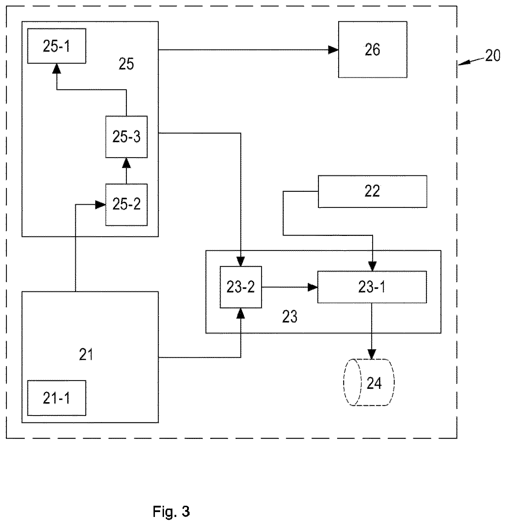

FIG. 3 is a block diagram for explaining a portable aerosol generating device according to another embodiment of the present invention. Referring to FIG. 3, the portable aerosol generating device 20 according to another embodiment of the present invention includes a heater 24 for generating heat as it is supplied with current, a battery 22 which can supply instantaneous high power to the heater 24, a heater control circuit 23 for regulating power supplied from the battery 22 to the heater 24, a microcontroller 21 for controlling the operation of the heater control circuit 23 for a heating time, and an error control means 25 provided separately from the microcontroller 21 and connected to both the microcontroller 21 and the heater control circuit 23 to receive and count a control signal being transferred from the microcontroller 21 to the heater control circuit 23 and simultaneously control the operation of the heater control circuit 23 depending on the counting result. The error control means 25 further includes a timer 25-1, a D/A converter 25-2 and a comparator 25-3. Also in some embodiments, the heater control circuit 23 includes a FET 23-1 connected between the battery 22 and the heater 24 and connected to the microcontroller 21 and a switching unit 23-2 connected between the microcontroller 21 and the FET 23-1 and connected to the error control means 25 to be turned on and off under the control of the error control means 25. When the heating starts, the microcontroller 21 forwards a control signal to the heater control circuit 23, and power supplied from the battery 22 is regulated in the heater control circuit 23 and supplied to the heater 24. The D/A converter 25-2 provided in the error control means 25 receives a control signal based on the timer 21-1 of the microcontroller 21 and converts the control signal into an analog signal, the analog signal converted in the D/A converter 25-2 is input to the comparator 25-3 and compared with a reference value in the comparator 25-3, and an output signal is sent to the timer 25-1. For example, the control signal based on the timer 21-1 of the microcontroller 21 is a PWM signal, the D/A converter 25-2 provided in the error control means 25 converts the PWM signal input from the microcontroller 21 into an analog signal and inputs the analog signal to the comparator 25-3, and the comparator 25-3 outputs a high signal to the timer 25-1 when receiving a signal having a value larger than the reference value and outputs a low signal to the timer 25-1 when receiving a signal having a value smaller than the reference value. The timer 25-1 counts, for example, a high signal input time, and the error control means 25 determines whether the preset heating time has been completed. If the high signal is not input for the preset heating time, the error control means 25 sends a control signal to the FET 23-1 through the switching unit 23-2 of the heater control circuit 23 to perform the heating operation for the preset heating time. In addition, if the high signal is input from the comparator 25-3 beyond the preset heating time, the error control means 25 sends a control signal to the switching unit 23-2 of the heater control circuit 23 to turn off the switching unit 23-2 to stop the heating operation. In some embodiments, the portable aerosol generating device includes a power supply unit 26 turned on and off under the control of the error control means 25, and the error control means 25 may stop the heating operation by controlling the power supply unit 26. During the heating operation, the duty of the PWM signal is changed to maintain the varying temperature. In some embodiments, the error control means 25 compares a duty duration of the high signal counted in the timer 25-1 with the preset time and if it is abnormal, the error control means 25 determines that a duty duration of the PWM signal is abnormal. In turn, the error control means 25 sends a control signal to the switching unit 23-2 of the heater control circuit 23 to turn off the switching unit 23-2 to stop the heating operation. In some embodiments, the respective timers 21-1 and 25-1 may adopt the respective counters. In some embodiments, the portable aerosol generating device includes a power supply unit 26 turned on and off under the control of the error control means 25, and the error control means 25 may stop the heating operation by controlling the power supply unit 26.

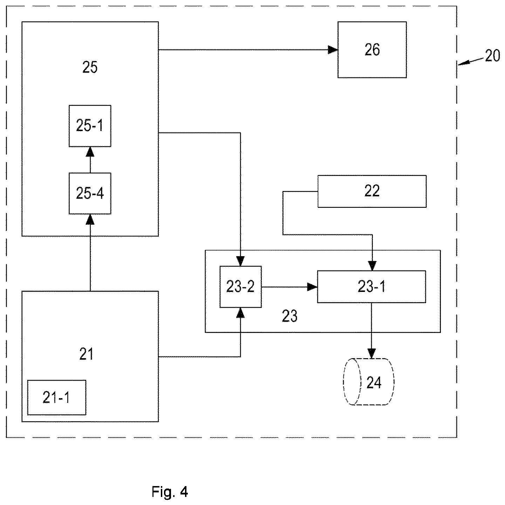

FIG. 4 is a block diagram for explaining a portable aerosol generating device according to a further embodiment of the present invention. Referring to FIG. 4, the portable aerosol generating device 20 according to the further embodiment of the present invention includes a heater 24 for generating heat as it is supplied with current, a battery 22 which can supply instantaneous high power to the heater 24, a heater control circuit 23 for regulating power supplied from the battery 22 to the heater 24, a microcontroller 21 for controlling the operation of the heater control circuit 23 for a heating time, and an error control means 25 provided separately from the microcontroller 21 and connected to both the microcontroller 21 and the heater control circuit 23 to receive and count a control signal being transferred from the microcontroller 21 to the heater control circuit 23 and simultaneously control the operation of the heater control circuit 23 depending on the counting result. The error control means 25 further includes a timer 25-1 and a frequency counter 25-4. Also in some embodiments, the heater control circuit 23 includes a FET 23-1 connected between the battery 22 and the heater 24 and connected to the microcontroller 21 and a switching unit 23-2 connected between the microcontroller 21 and the FET 23-1 and connected to the error control means 25 to be turned on and off under the control of the error control means 25. When the heating starts, the microcontroller 21 forwards a control signal to the heater control circuit 23, and power supplied from the battery 22 is regulated in the heater control circuit 23 and supplied to the heater 24. The frequency counter 25-4 provided in the error control means 25 receives a control signal based on the timer 21-1 of the microcontroller 21 and counts its frequency, and the timer 25-1 counts the counting time of the frequency counter 25-4. For example, the control signal based on the timer 21-1 of the microcontroller 21 is a PWM signal, the frequency counter 25-4 provided in the error control means 25 counts a frequency of the PWM signal input from the microcontroller 21, and the timer 25-1 counts the time for which the frequency counter 25-4 counts the frequency of the PWM signal. Then, the error control means 25 compares the counted time with the preset time and if it is determined that the counting time of the frequency of the PWM signal is abnormal, the error control means 25 sends a control signal to the switching unit 23-2 of the heater control circuit 23 to turn off the switching unit 23-2 to stop the heating operation. In some embodiments, the portable aerosol generating device includes a power supply unit 26 turned on and off under the control of the error control means 25, and the error control means 25 may stop the heating operation by controlling the power supply unit 26. In addition, the error control means 25 determines whether the preset heating time has been completed. If the frequency counter 25-4 does not count the PWM signal for the preset heating time, the error control means 25 sends a control signal to the FET 23-1 through the switching unit 23-2 of the heater control circuit 23 to perform the heating operation for the preset heating time. In addition, if the frequency counter 25-4 counts the frequency of the PWM signal beyond the preset heating time, the error control means 25 sends a control signal to the switching unit 23-2 of the heater control circuit 23 to turn off the switching unit 23-2 to stop the heating operation. In some embodiments, the portable aerosol generating device includes a power supply unit 26 turned on and off under the control of the error control means 25, and the error control means 25 may stop the heating operation by controlling the power supply unit 26.

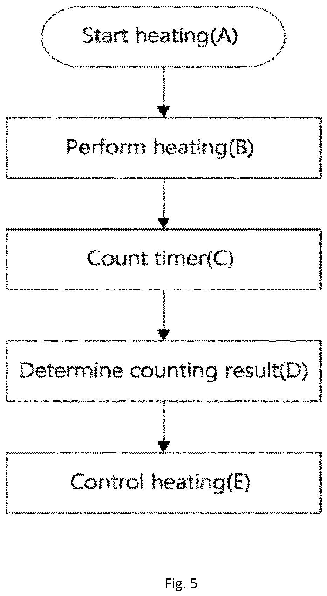

FIG. 5 is a flowchart for explaining an operation method of a portable aerosol generating device according to the present invention. Referring to FIG. 5, the operation method of the portable aerosol generating device according to the present invention includes the steps of: starting heating; performing the heating; counting a control signal based on a timer of a microcontroller; determining the counting result; and controlling the heating depending on the counting result. In step A, when the heating starts, in step B, the microcontroller 21 sends a control signal based on the timer 21-1, for example, a PWM signal to the heater control circuit 23 to perform the heating operation. In step C, the error control means 25 receives the control signal based on the timer 21-1 of the microcontroller 21, for example, a PWM signal, and counts its frequency. In step D, the error control means 25 determines the counting result, for example, determines whether the heating time has been completed. If the heating time has been completed, in step E, the error control means 25 stops the heating operation by controlling the heater control circuit 23, for example, stops the heating operation by turning off the switching unit 23-2 of the heater control circuit 23 or by turning off the power supply unit 26. If the PWM signal is not counted even though the heating time has not been completed, the error control means 25 proceeds the heating operation by controlling the heater control circuit 23, for example, sends a control signal to the FET 23-1 through the switching unit 23-2 of the heater control circuit 23 to perform the heating operation for the preset heating time.

Also in some embodiments, in step D, when determining the counting result, the error control means 25 compares a duty duration of the control signal based on the timer 21-1 of the microcontroller 21, for example, the PWM signal with the preset time. If it is determined that the duty duration is abnormal, in step E, the error control means 25 stops the heating operation by turning off the switching unit 23-2 of the heater control circuit 23 or by turning off the power supply unit.

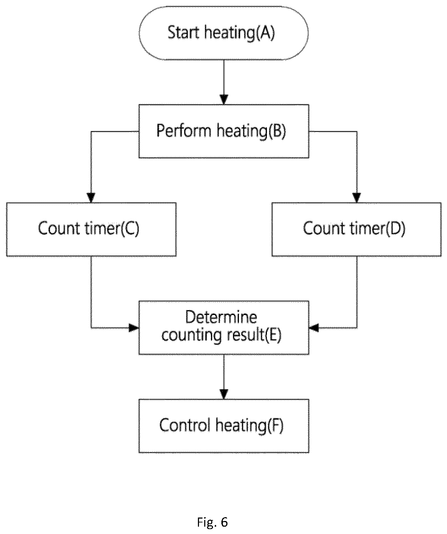

FIG. 6 is another flowchart for explaining an operation method of a portable aerosol generating device according to the present invention. Referring to FIG. 6, the operation method of the portable aerosol generating device according to the present invention further includes a step of counting a control signal based on a timer of an error control means, in combination with the step of counting the control signal based on the timer of the microcontroller. As shown in FIG. 6, in step A, when the heating starts, in step B, the microcontroller 21 sends a control signal based on the timer 21-1, for example, a PWM signal to the heater control circuit 23 to perform the heating operation. In step C, the error control means 25 receives the control signal based on the timer 21-1 of the microcontroller 21, for example, the PWM signal, and counts its frequency, and in combination with this step, in step D, the error control means 25 counts a frequency of a control signal based on the timer 25-1 of the error control means 25. In step E, the error control means 25 compares the counting result of the PWM signal received from the microcontroller 21 with the counting result of the frequency of the control signal based on the timer 25-1 of the error control means 25, for example, determines whether the heating time has been completed. If it is determined that the heating time has been completed by counting the PWM signal received from the microcontroller 21 for the preset heating time or counting the frequency of the control signal based on the timer 25-1 of the error control means 25, the error control means 25 stops the heating operation by controlling the heater control circuit 23, for example, stops the heating operation by turning off the switching unit 23-2 of the heater control circuit 23 or by turning off the power supply unit 26. If the PWM signal from the microcontroller 21 is not counted even though the heating time has not been completed, the error control means 25 proceeds the heating operation by controlling the heater control circuit 23, for example, sends a control signal to the FET 23-1 through the switching unit 23-2 of the heater control circuit 23 to perform the heating operation for the preset heating time.

While the present invention has been illustrated and described in connection with the accompanying drawings and the preferred embodiments, the present invention is not limited thereto and is defined by the appended claims. Therefore, it will be understood by one of ordinary skill in the art that various modifications and changes can be made thereto without departing from the spirit and scope of the invention defined by the appended claims.

The present invention makes it possible to provide a portable aerosol generating device capable of preventing overheating of a heater by controlling a heating time by including an error control means provided separately from a microcontroller.

In addition, the present invention makes it possible to provide a portable aerosol generating device capable of preventing overheating of a heater and/or equipment failure by including an error control means provided separately from the microcontroller to determine a PWM signal of the microcontroller and sense a malfunction thereof.

## Claims

1. A portable aerosol generating device comprising:
a heater for generating heat as it is supplied with current;
a battery which can supply instantaneous high power to the heater;
a heater control circuit for regulating power supplied from the battery to the heater;
a microcontroller for controlling the operation of the heater control circuit for a heating time; and
an error control means provided separately from the microcontroller and connected to both the microcontroller and the heater control circuit to receive and count a control signal being transferred from the microcontroller to the heater control circuit and simultaneously control the operation of the heater control circuit depending on the counting result.

2. The portable aerosol generating device of claim 1, wherein the microcontroller and the error control means comprise timers, respectively, and the error control means receives a control signal based on the timer of the microcontroller, compares the control signal with a control signal based on the timer of the error control means, and sends to the heater control circuit the control signal based on the timer independently from the microcontroller.

3. The portable aerosol generating device of claim 1, wherein the error control means comprises a D/A converter for receiving a control signal transferred from the microcontroller to the heater control circuit and converting the control signal into an analog signal, a comparator for comparing the signal output from the D/A converter with a reference value and forwarding an output signal, and a timer for counting the output signal forwarded from the comparator.

4. The portable aerosol generating device of claim 1, wherein the error control means comprises a frequency counter for receiving a control signal transferred from the microcontroller to the heater control circuit and counting its frequency and a timer for counting a count time of the frequency counter.

5. The portable aerosol generating device of any one of claims 1 to 4, wherein the control signal based on the timer of the microcontroller is a PWM signal transferred from the microcontroller to the heater control circuit.

6. The portable aerosol generating device of claim 5, wherein the heater control circuit comprises a FET connected between the battery and the heater and connected to the microcontroller and a switching unit connected between the microcontroller and the FET and connected to the error control means to be turned on and off under the control of the error control means.

7. The portable aerosol generating device of claim 5, comprising a power supply unit turned on and off under the control of the error control means.

8. An operation method of a portable aerosol generating device, the operation method comprising the steps of:
starting heating;
performing the heating;
counting a control signal based on a timer of a microcontroller;
determining the counting result; and
controlling the heating depending on the counting result.

9. The operation method of claim 8, further comprising a step of counting a control signal based on a timer of an error control means, in combination with the step of counting the control signal based on the timer of the microcontroller.

10. The operation method of claim 9, wherein, in the step of counting the control signal based on the timer of the microcontroller, the control signal based on the timer of the microcontroller is a PWM signal and a frequency of the PWM signal is counted.

11. The operation method of claim 9, wherein, in the step of determining the counting result, whether the heating time has been completed is determined.

12. The operation method of claim 8, wherein, in the step of counting the control signal based on the timer of the microcontroller, the control signal based on the timer of the microcontroller is a PWM signal and a duty duration of the PWM signal is counted, and in the step of determining the counting result, the duty duration is compared with the preset time.

13. The operation method of any one of claims 8 to 12, wherein, in the step of controlling the heating depending on the counting result, a switching unit of a heater control circuit is turned on and off.

14. The operation method of any one of claims 8 to 12, wherein, in the step of controlling the heating depending on the counting result, a power supply unit is turned on and off.
